# EUROPÄISCHE PATENTSCHRIFT

(11) **EP 0 559 687 B1**
(45) Veröffentlichungstag und Bekanntmachung des Hinweises auf die Patenterteilung: **24.01.2001**
(21) Anmeldenummer: 91920302.6
(22) Anmeldetag: 16.11.1991
(51) Int. Cl.: C07D 209/48, C07C 229/44

(54) **2-AMINO-ZIMTSÄUREESTER**
2-AMINOCINNAMIC ACID ESTERS
ESTERS D'ACIDE 2-AMINOCINNAMIQUE

(30) Priorität: 28.11.1990 DE 4037840
(43) Veröffentlichungstag der Anmeldung: 15.09.1993
(73) Patentinhaber: BASF Aktiengesellschaft, 67063 Ludwigshafen (DE)
(72) Erfinder: RUEB, Lothar, D-6720 Speyer (DE); EICKEN, Karl, D-6706 Wachenheim (DE); SCHAEFER, Bernd, D-6749 Dierbach (DE); REISSENWEBER, Gernot, D-6737 Boehl-Iggelheim (DE); SCHAEFER, Peter, D-6702 Bad Duerkheim (DE)
(86) Internationale Anmeldenummer: EP9102166
(87) Internationale Veröffentlichungsnummer: WO9209575

(56) Entgegenhaltungen:
- EP-A- 0 300 387
- DE-A- 3 904 082
- J. Am. Chem. Soc., Band 66, 1944, E.R. BLOUT et al.: "The catalytic reduction of nitrocinnamic acids and esters", Seiten 1442-1443, siehe das ganze Dokument
- Patent Abstracts of Japan, Band 9, Nr 1, C259, Zusammenfassung von JP 59-155358, publ 1984-09-04

## Beschreibung

Die vorliegende Erfindung betrifft ein verbessertes Verfahren zur Herstellung von 3-(3,4,5,6-Tetrahydrophthalimido)-zimtsäureestern der allgemeinen Formel I 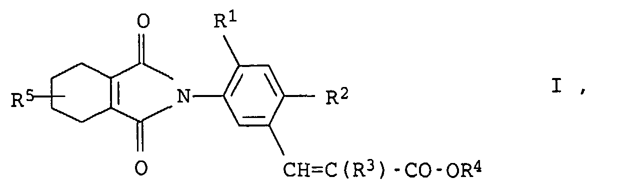 in der R¹ Wasserstoff oder Halogen, R² und R³ Halogen, R⁴ Wasserstoff; C₁-C₄-Alkyl, wobei die Alkylgruppe noch eine oder zwei C₁-C₄-Alkoxy und/oder C₁-C₄-Alkylthioreste tragen kann; C₃-C₇-Cycloalkyl; C₃-C₆-Alkenyl; C₃-C₆-Alkinyl oder Benzyl und R⁵ Wasserstoff oder Methyl bedeuten.

Aus DE-A 3 904 082 ist bekannt, daß 3-(3,4,5,6-Tetrahydrophthalimido)-zimtsäureester durch Umsetzung eines 3-(3,4,5,6-Tetrahydrophthalimido)-benzaldehyds mit einem entsprechend substituierten Phosphorylid erhalten werden können.

Es ist ebenso allgemein bekannt, Nitrozimtsäureester, die jedoch kein alpha-ständiges Halogenatom tragen, mittels Eisen in Essigsäure (JP-A 155 358/84) oder Wasserstoff an einem Palladium-, Platin- oder Raney-Nickel-Katalysator (EP-A 345 637 und DE-A 39 31 615) zu Aminozimtsäureestern zu reduzieren und diese in Tetrahydrophthalimido-zimtsäureester vom Typ der Verbindung I zu überführen.

Auch EP-A 300 387 lehrt die Darstellung von Anilinen mit Alkenylcarbonsäureester-Seitenkette aus den entsprechenden Nitro-Verbindungen mittels Reduktion mit Eisen, welches in stöchiometrischen Mengen eingesetzt wird.

Gemäß der Lehre der Bull. Chem. Soc. Jap. 49, 2284 (1976), der BE-A 855-464 und der EP-A 240 659 wird jedoch bei Verwendung von Wasserstoff als Reduktionsmittel auch die ungesättigte Seitenkette eines Aromaten mit hydriert. Aus der EP-A 240 659 ist weiter zu entnehmen, daß diese Nebenreaktion bei Verwendung von Platinoxid als Katalysator oder bei Übergang zu Metallen wie Eisen als Reduktionsmittel nicht auftritt.

Zusätzlich ist aus der EP-A 369 212 und aus Chimia 41, 73 (1987) bekannt, daß mit Wasserstoff in Gegenwart von Katalysatoren wie Palladium, Platin und Raney-Nickel katalytische Dehalogenierungen möglich sind, was in diesen Druckschriften sogar für präparative Synthesen genutzt wird.

Zur Vermeidung der genannten Konkurrenzreaktionen wird deshalb in der älteren deutschen Anmeldung DE-A 39 31 615 vorgeschlagen, speziell die 3-Nitrozimtsäureester II, die ein alpha-ständiges Chlor- oder Bromatom tragen, nur mit milden Reduktionsmitteln wie Zinn(II)-Salzen oder mit Eisen zu den 3-Aminoestern III zu reduzieren.

Nachteilig bei dieser Methode ist jedoch die großtechnisch problematische Abtrennung und Entsorgung der bei der Reduktion als Nebenprodukte entstehenden Zinn- und Eisensalze.

Der Erfindung lag daher die Aufgabe zugrunde, die Verbindung I besser zugänglich zu machen.

Demgemäß wurde ein Verfahren zur Herstellung von 3-(3,4,5,6-Tetrahydrophthalimido)-zimtsäureestern der Formel I gefunden, welches dadurch gekennzeichnet ist, daß man einen 3-Nitro-zimtsäureester der allgemeinen Formel II 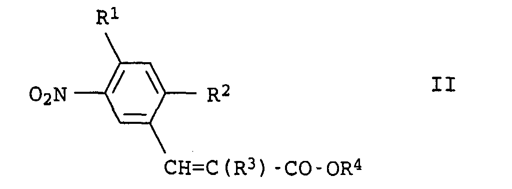 mit Wasserstoff in Gegenwart eines Katalysators reduziert und die erhaltenen 3-Amino-zimtsäureester der Formel III 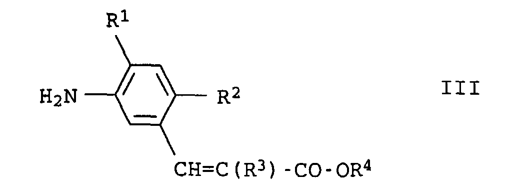 anschließend oder gleichzeitig mit einem 3,4,5,6-Tetrahydrophthalsäureanhydrid der allgemeinen Formel IV 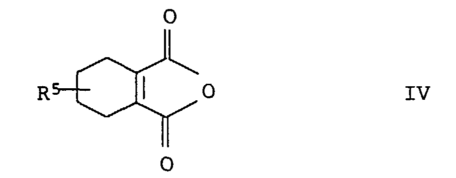 kondensiert.

Die als Ausgangsstoffe dienenden 3-Nitro-zimtsäureester II sind auf verschiedene Weise darstellbar, und zwar bevorzugt nach einer der folgenden Methoden:
a) Umsetzung von m-Nitroaldehyden IV mit Phosphoryliden V 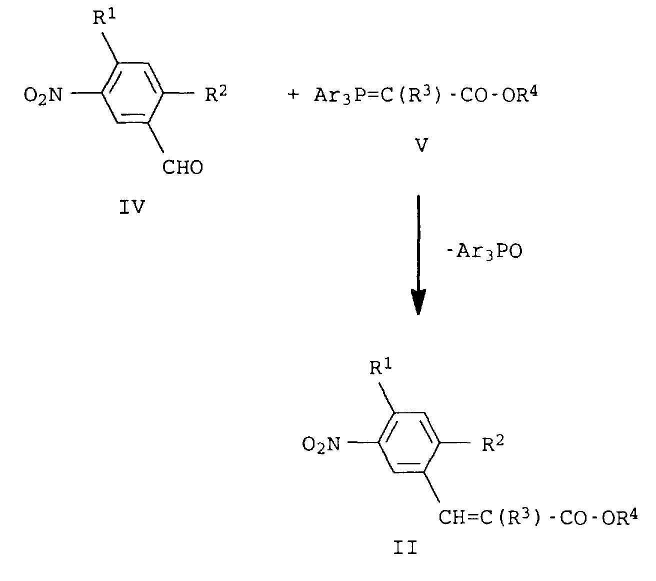
   Die Reste Ar können gleich oder verschieden sein und bedeuten C-organische Reste, insbesondere Phenyl.
   Die Umsetzung erfolgt im allgemeinen nach an sich bekannten Methoden (vgl. EP-A 345 637) in einem inerten Lösungs- oder Verdünnungsmittel, z.B. Alkoholen, vorzugsweise in dem Alkohol R⁴-OH, oder in chlorierten Kohlenwasserstoffen wie Dichlormethan.
   Bevorzugt setzt man alle Ausgangsverbindungen in etwa stöchiometrischem Verhältnis ein, jedoch kann in manchen Fällen auch ein Überschuß der einen oder anderen Komponente, etwa bis zu 10 mol-%, empfehlenswert sein.
   Normalerweise liegt die Reaktionstemperatur zwischen 0°C und der Siedetemperatur des jeweiligen Lösungsmittels.
   Besondere Bedingungen bezüglich des Druckes sind nicht erforderlich, zweckmäßigerweise arbeitet man deshalb bei Normaldruck.
   Die Ausgangsverbindungen IV sind bekannt. Die Phosphorylide V können nach an sich bekannten Methoden [z.B. Chem. Ber. 95, 3003 (1962)] dargestellt werden.,
(b) Nitrierung von Zimtsäureestern VI 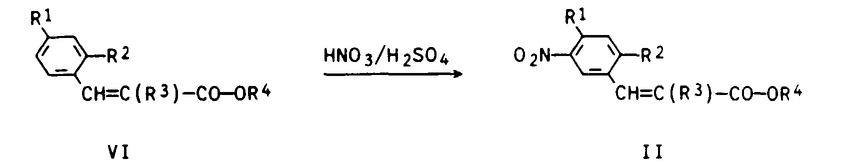
   Die Umsetzung erfolgt nach an sich bekannten Methoden (vgl. auch JP-A 155 358/84), gewünschtenfalls in einem inerten Lösungs- oder Verdünnungsmittel, bei Temperaturen zwischen (-10) und 50°C.
   Die Menge an Nitrierreagenz ist nicht kritisch. Bevorzugt arbeitet man mit einem Überschuß an Nitrierreagenz in Schwefelsäure oder lösungsmittelfrei in Salpetersäure.
   Bezüglich des Druckes gelten die Angaben für Methode (a).
   Die Ausgangsverbindungen sind analog Methode (a) herstellbar.
(c) Halogenierung von m-Nitrozimtsäureestern VII 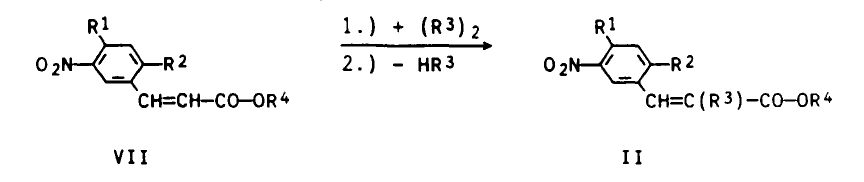
   Die Umsetzung erfolgt im allgemeinen in an sich bekannter Weise (vgl. EP-A 240 659) in einem inerten Lösungs- bzw. Verdünnungsmittel, z.B. einem Halogenkohlenwasserstoff wie Methylenchlorid, Chloroform, Tetrachlormethan, 1,1,1-Trichlorethan und Chlorbenzol.

Bevorzugt arbeitet man mit einem Überschuß an Halogen (R³)₂ bis etwa 10 mol-%, bezogen auf die Menge an VI.

Normalerweise liegt die Reaktionstemperatur zwischen 0°C und der Siedetemperatur des jeweiligen Lösungsmittels, insbesondere zwischen 15 und 40°C.

Auch hierbei empfiehlt sich das Arbeiten bei Normaldruck.

Im Falle der Chlorierung der m-Nitrozimtsäureester VII besteht eine besonders vorteilhafte Ausführungsform der Methode (c) darin, den m-Nitrozimtsäureester VII in Gegenwart einer Lewis-Säure zu chlorieren und den hierbei entstehenden α,β-Dichlor-β-(3-nitrophenyl)-propionsäureester (als Diastereoisomerengemisch) einer Chlorwasserstoff-Eliminierung zu unterwerfen.

Geeignete Lewissäuren sind insbesondere Übergangsmetallhalogenide wie Zink(II)chlorid, Eisen(III)chlorid und Aluminiumchlorid.

Als Lösungsmittel kommen vorzugsweise chlorierte Kohlenwasserstoffe wie Dichlormethan und 1,2-Dichlorbenzol in Betracht.

Die Menge an Lewis-Säure ist nicht kritisch; im allgemeinen verwendet man 2 bis 200 mol-%, vorzugsweise 5 bis 140 mol-%, an Lewis-Säure, bezogen auf die Menge an m-Nitrozimtsäureester VII.

Zur Vermeidung langer Reaktionszeiten empfiehlt sich eine Temperatur zwischen 20°C und der Siedetemperatur des Reaktionsgemisches, insbesondere zwischen 50 und 90°C.

Die anschließende Chlorwasserstoff-Eliminierung erfolgt in Gegenwart einer Hilfsbase, vorzugsweise ohne Zu- oder Abführung von Energie.

Durch Wahl der Hilfsbase läßt sich die Stereochemie der Eliminierung steuern, so daß überwiegend das E- oder Z-Isomere der 3-Nitro-zimtsäureester II erhalten wird.

Als Hilfsbasen kommen beispielsweise die Erdalkalimetallsalze von organischen Säuren oder organische Amine in Betracht. Ganz besonders bevorzugt arbeitet man mit Natriumacetat in Eisessig oder mit Triethylamin in Methylenchlorid, wobei überwiegend das Z-Isomer der 3-Nitro-zimtsäureester II entsteht.

Bevorzugt setzt man den α,β-Dichlor-β-(3-nitrophenyl)-propionsäureester und die Base in stöchiometrischem Verhältnis ein, oder man arbeitet mit einem geringen Überschuß an Base bis etwa 10 mol-%.

Die Ausgangsverbindungen VII sind analog Methode (a) oder (b) zugänglich.
(d) Umsetzung von Nitrozimtsäurechloriden VIII mit Alkoholen IX

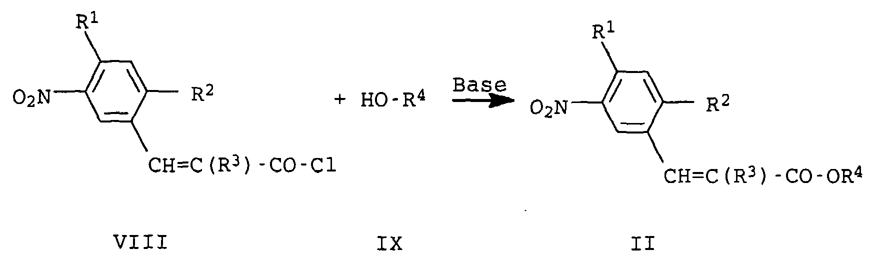

Die Umsetzung erfolgt im allgemeinen in an sich bekannter Weise (vgl. Houben-Weyl, Methoden der Organischen Chemie, Band X/2, 747) in einem inerten Lösungs- oder Verdünnungsmittel, vorteilhaft in Anwesenheit einer Base.

Als Lösungs- oder Verdünnungsmittel eignen sich insbesondere höhersiedende Kohlenwasserstoffe wie o-, m-, p-Xylol und Toluol, Ester wie Ethylacetat und Ether wie Dioxan und Tetrahydrofuran.

Als Basen kommen vorzugsweise tertiäre Amine wie Triethylamin und Pyridin sowie anorganische Salze, z.B. Alkalimetallhydroxide wie Natrium- und Kaliumhydroxid und Alkalimetallcarbonate wie Natriumcarbonat, in Betracht.

Normalerweise liegt die Reaktionstemperatur zwischen (-10) und 200°C, insbesondere zwischen 0 und 150°C.

Bezüglich der Mengenverhältnisse und des Druckes gelten die Angaben für Methode (a).

Die Zimtsäurechloride VIII sind nach an sich bekannten Methoden erhältlich (vgl. EP-A-240 659, Beispiel 8).

Erfindungsgemäß werden die Nitrozimtsäurederivate II mittels Wasserstoff in Gegenwart eines Metallkatalysators, z.B. Palladium, Platin und Nickel, zu den entsprechenden Aminozimtsäurederivaten III reduziert.

Die Reduktion erfolgt zweckmäßigerweise in einem inerten polaren Lösungs- oder Verdünnungsmittel, beispielsweise einem Ether wie Tetrahydrofuran, einem Amid wie Dimethylformamid, einer kurzkettigen Carbonsäuren wie Essig- und Propionsäure, dem Ester einer kurzkettigen Carbonsäure wie Essigsäureethylester, oder in dem Alkohol HO-R⁴, insbersondere in Methanol oder Ethanol.

Die Menge an Katalysator ist nicht kritisch; normalerweise verwendet man 1 bis 50 mol-% an Katalysator, bezogen auf die Menge an Nitrozimtsäurederivat II.

Zweckmäßig führt man die Hydrierung bei einem Wasserstoffdruck zwischen 1 und 100 bar, bevorzugt zwischen 1 und 10 bar, durch.

Die Reaktionstemperatur liegt im allgemeinen zwischen 0°C und dem Siedepunkt des jeweiligen Lösungsmittel.

Das Verfahren kann sowohl diskontinuierlich als auch kontinuierlich durchgeführt werden. Bei der kontinuierlichen Arbeitsweise leitet man das Nitrozimtsäurederivat in einer mit Wasserstoff gesättigten Lösung vorzugsweise über ein Festbett, das mit dem Katalysator belegt wurde.

In einer bevorzugten Ausführungsform wird zu einer Mischung aus Nitrozimtsäurederivat II, Verdünnungsmittel und Katalysator so lange Wasserstoff zudosiert, bis kein Verbrauch an Wasserstoff mehr festzustellen ist.

Die Aufarbeitung des Reaktionsgemisches erfolgt wie üblich, so daß sich nähere Ausführungen hierzu erübrigen.

Die erhaltenen 3-Amino-zimtsäurederivate III werden anschließend durch Kondensation mit 3,4,5,6-Tetrahydrophthalsäureanhydriden der Formel IV in die 3-(3,4,5,6-Tetrahydrophthalimido)-zimtsäureester I überführt: 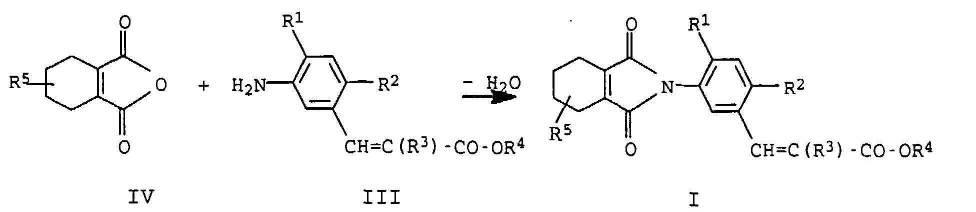 Die Umsetzung erfolgt normalerweise in einem inerten aprotischen Lösungsmittel bei einer Reaktionstemperatur zwischen 20°C und der Siedetemperatur des Lösungsmittels, insbesondere zwischen 40 und 140°C.

Als Lösungsmittel eignen sich niedere Alkansäuren wie Eisessig, Propionsäure und Isobuttersäure, die Ester der genannten Säuren wie Essigsäureethylester, aromatische Kohlenwasserstoffe wie Toluol und o-, m-, p-Xylol sowie aprotische Lösungsmittel wie Dimethyl- und Diethylformamid. Beim Arbeiten in einem aprotischen Lösungsmittel empfiehlt es sich, das entstehende Wasser laufend zu entfernen.

Zweckmäßig setzt man die Ausgangsverbindungen III und IV in stöchiometrischem Verhältnis ein, jedoch kann in manchen Fällen auch ein Überschuß der einen oder anderen Komponente, etwa bis zu 10 mol-%, empfehlenswert sein.

Vorzugsweise arbeitet man bei Atmosphärendruck oder unter dem Eigendruck des jeweiligen Lösungsmittels. Geringerer oder höherer Druck ist möglich, bringt aber im allgemeinen keine Vorteile.

Eine besonders vorteilhafte Variante des erfindungsgemäßen Verfahrens besteht darin, die durch Reduktion der 3-Nitro-zimtsäurederivate II erhaltenen Verfahrensprodukte III ohne Isolierung aus der Reaktionsmischung mit den 3,4,5,6-Tetrahydrophthalsäureanhydriden IV umzusetzen. Bei dieser Verfahrensweise kann das 3,4,5,6-Tetrahydrophthalsäureanhydrid IV vor oder nach der Hydrierung in die Reaktionsmischung eingebracht werden. Bevorzugt arbeitet man hierbei in einer niederen Alkansäure, insbesondere in Propionsäure oder in einem aprotischen Lösungsmittel, insbesondere in einem Ether wie Tetrahydrofuran oder einem Amid wie Dimethylformamid.

Das erfindungsgemäße Verfahren läßt sich mit Erfolg zur Synthese aller definitionsgemäßen 3-(3,4,5,6-Tetrahydrophthalimido)-zimtsäurederivate I mit α-ständigem Chlor- oder Bromatom anwenden, vor allem auf solche Verbindungen, bei denen die Substituenten die folgende Bedeutung haben:
- R¹ -: Wasserstoff oder Halogen wie Fluor, Chlor, Brom oder Iod,insbesondere Wasserstoff oder Fluor;
- R² -: Halogen wie vorstehend genannt, insbesondere Chlor;
- R³ -: Chlor oder Brom;
- R⁴ -: Wasserstoff;
- C₁-C₄-Alkyl wie Methyl, Ethyl, n-Propyl, Isopropyl, n-Butyl und tert.-Butyl, wobei die Alkylgruppe noch eine oder zwei C₁-C₄-Alkoxyreste wie Methoxy, Ethoxy, n-Propoxy, Isopropoxy, n-Butoxy und tert.-Butoxy, insbesondere Methoxy und Ethoxy, und/oder C₁-C₄-Alkylthioreste wie Methylthio, Ethylthio, n-Propylthio, Isopropylthio, n-Butylthio und tert.-Butylthio, insbesondere Methylthio, tragen kann;
- C₃-C₇-Cycloalkyl wie Cyclopropyl, Cyclobutyl, Cyclopentyl und Cyclohexyl;
- C₃-C₆-Alkenyl wie 2-Propenyl, 2-Butenyl, 3-Butenyl; 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, l,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl;
- C₃-C₆-Alkinyl wie 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
- Benzyl;
- R⁵: Wasserstoff oder Methyl.

3-(3,4,5,6-Tetrahydrophthalimido)-zimtsäureester I finden im Pflanzenschutz insbesondere als Herbizide (vorzugsweise in Getreide) und als Abszissionsmittel (in Baumwolle) Verwendung.

### Herstellungsbeispiele

(E/Z)-N-[3-(2-chlor-2-methoxycarbonylethen-1-yl)-4-chlorphenyl]-3,4,5,6-tetrahydrophthalimid

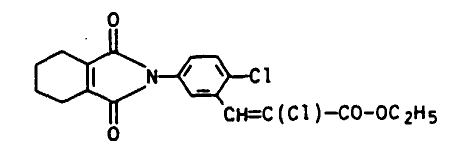

### Beispiel 1

Zu einer Lösung aus 780 g (3 mol) (E/Z)-2-Chlor-5-amino-alpha-chlor-zimtsäureethylester in 7 l Propionsäure wurden bei 20 bis 25°C portionsweise 456 g (3 mol) 3,4,5,6-Tetrahydrophthalsäureanhydrid gegeben. Die entstandene klare Lösung wurde 5 Stunden bei 60°C gerührt, wobei das Produkt auszukristallisieren begann. Nach Abkühlung auf ca. 25°C verdünnte man mit 4 l Wasser und rührte die Mischung noch gut 14 Stunden. Anschließend wurde das Produkt abgetrennt, mit 4,5 l Wasser gewaschen und getrocknet. Ausbeute: 80 % (Reinheit ca. 98 % nach HPLC); Fp.: 111-112°C.

### Vorstufe 1 α

(E/Z)-2-Chlor-5-nitro-alpha-chlor-zimtsäureethylester 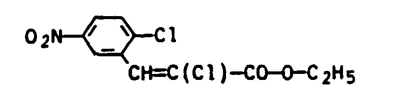 nach Methode (a):
Zu einer Lösung aus 37 g (0,2 mol) 2-Chlor-5-nitrobenzaldehyd in 350 ml Ethanol wurden 84,1 g (0,22 mol) Ethoxycarbonyl-chlormethylentriphenylphosphoran gegeben. Man rührte das Gemisch eine Stunde bei 25°C, wonach das Produkt abfiltriert und mit Petrolether gewaschen wurde.
Ausbeute: 70 %; Fp.: 97-98°C.
nach Methode (c):
Zu einer Lösung aus 10 g (39 mmol) 2-Chlor-5-nitro-zimtsäureethylester in 150 ml 1,2-Dichlorbenzol wurden 0,64 g (4 mmol) wasserfreies Eisen(III)chlorid gegeben, wonach man bei 100°C 3 Stunden lang Chlor eingaste. Anschließend wurde das Reaktionsgemisch mit gesättigter Natriumhydrogencarbonat-Lösung und mit Wasser gewaschen, über Natriumsulfat getrocknet und eingeengt.
Ausbeute: 75 % α,β-Dichlor-β-(2-Chlor-5-nitrophenyl)-propionsäureethylester (3:1-Diastereomerengemisch); Fp: 63-65°C

1 g (3 mmol) des vorstehend erhaltenen α,β-Dichlor-β-(2-chlor-5-nitrophenyl)-propionsäureethylesters wurden in 30 ml Methylenchlorid gelöst, wonach man die Lösung mit 0,31 g (3 mmol) Triethylamin versetzte. Die Mischung wurde ca. 15 Stunden bei 20-25°C gerührt, danach zweimal mit Wasser gewaschen, mit Natriumsulfat getrocknet und unter reduziertem Druck eingeengt.
Ausbeute: 100 % eines Rohproduktes, das noch 5 bis 10 % der Dichlorverbindung enthielt.

### Vorstufe 1 β

(E/Z)-2-Chlor-5-amino-alpha-chlor-zimtsäureethylester 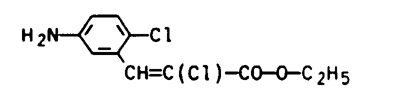 Eine Suspension aus 5,8 g (20 mmol) (E/Z)-2-Chlor-5-nitro-alpha-chlor-zimtsäureethylester in 150 ml Ethanol wurde mit 3 g (51 mmol) Raney-Nickel versetzt. Nach Aufpressen von 1,05 bar Wasserstoff wurde die Mischung bei 30°C bis zur Beendigung der Wasserstoffaufnahme hydriert (ca. 7 Stunden). Anschließend trennte man den Katalysator ab, entfernte das Lösungsmittel und wusch den Rückstand mit Petrolether.
Ausbeute: 77 %; Fp.: 110-111°C.

### Beispiel 2 (einstufige Variante)

Eine Mischung aus 29 g (100 mmol) 5-Nitro-2-chlor-alpha-chlor-zimtsäurethylester, 3 g (51 mmol) Raney-Nickel, 15,2 g (100 mmol) 3,4,5,6-Tetrahydrophthalsäureanhydrid und 100 ml Propionsäure wurde bei 50 bis 60°C durch Aufpressen von 1,05 bar Wasserstoff bis zur Beendigung der Wasserstoffaufnahme hydriert (ca. 18 Stunden). Anschließend trennte man den Katalysator aus dem noch warmen Reaktionsgemisch ab und versetzte die erhaltene Lösung mit 100 ml Wasser. Nach 30 Minuten Rühren wurde das auskristallisierte Produkt abgetrennt und mit Wasser neutral gewaschen.
Ausbeute: 85 %; Fp.: 108-110°C.

## Patentansprüche

1. Verfahren zur Herstellung von 3-(3,4,5,6-Tetrahydrophthalimido)-zimtsäureestern der allgemeinen Formel I 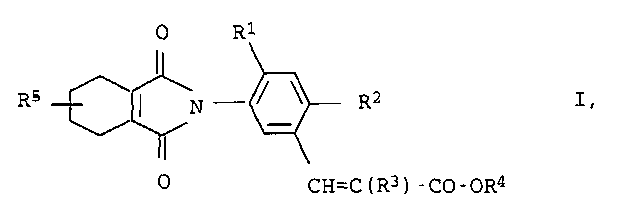 in der R¹ Wasserstoff oder Halogen, R² und R³ Halogen, R⁴ Wasserstoff; C₁-C₄-Alkyl, wobei die Alkylgruppe noch eine oder zwei C₁-C₄-Alkoxy und/oder C₁-C₄-Alkylthioreste tragen kann; C₃-C₇-Cycloalkyl; C₃-C₆-Alkenyl; C₃-C₆-Alkinyl oder Benzyl und R⁵ Wasserstoff oder Methyl bedeuten, indem man einen 3-Nitrozimtsäureester der allgemeinen Formel II 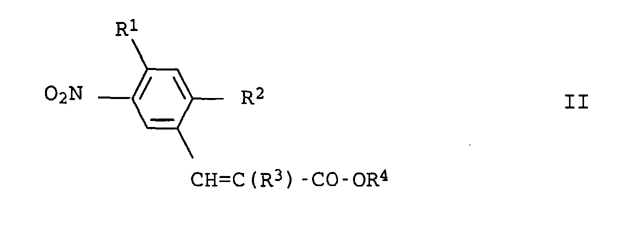 zu einem 3-Amino-zimtsäureester der Formel III 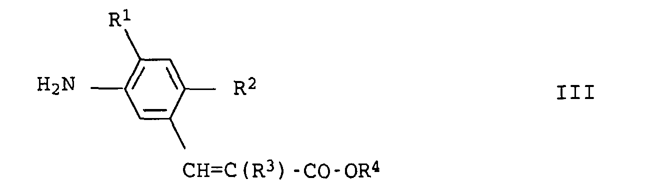 reduziert und einen 3-Amino-zimtsäureester der Formel III mit einem 3,4,5,6-Tetrahydrophthalsäureanhydrid der allgemeinen Formel IV 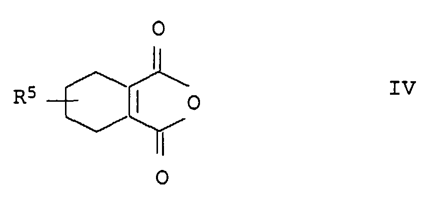 kondensiert, dadurch gekennzeichnet, daß die Reduktion des 3-Nitro-zimtsäureesters der allgemeinen Formel II zu dem 3-Amino-zimtsäureester der allgemeinen Formel III mit Wasserstoff in Gegenwart eines Katalysators aus der Gruppe Platin, Palladium und Nickel, durchgeführt wird und daß der 3-Aminozimtsäureester der allgemeinen Formel III anschließend oder gleichzeitig mit einem 3,4,5,6-Tetrahydrophthalsäureanhydrid der allgemeinen Formel IV kondensiert wird.

2. Verfahren nach Anspruch 1, dadurch gekennzeichnet, daß der 3-Amino-zimtsäureester der allgemeinen Formel III ohne Isolierung mit einem 3,4,5,6-Tetrahydrophthalsäureanhydrid der allgemeinen Formel IV kondensiert wird.

3. Verfahren nach Anspruch 1, dadurch gekennzeichnet, daß man die Reduktion in einem polaren Lösungsmittel vornimmt.

4. Verfahren nach Anspruch 1, dadurch gekennzeichnet, daß man hierbei von einem 3-Nitro-zimtsäureester II ausgeht, wie er bei der Herstellung von II durch Halogenierung eines m-Nitrozimtsäureesters der Formel VII 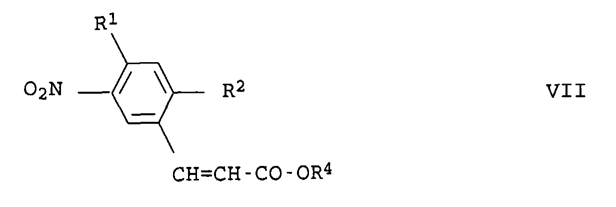 anfällt.

5. Verfahren nach Anspruch 1, dadurch gekennzeichnet, daß man hierbei von einem 3-Nitro-zimtsäureester II ausgeht, wie er bei der Umsetzung eines m-Nitroaldehyds X 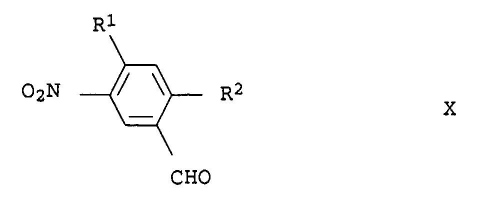 mit einem Phosphorylid V
Ar₃P=C(R³)-CO-OR⁴ V
anfällt.

## Claims

1. A process for preparing 3-(3,4,5,6-tetrahydrophthalimido)cinnamic esters of the general formula I 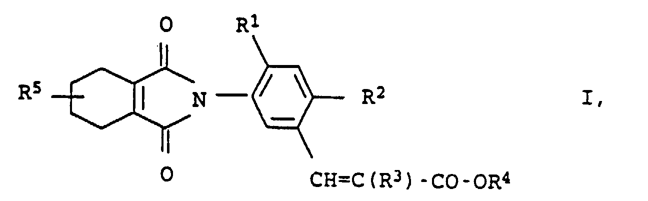 in which R¹ is hydrogen or halogen, R² and R³ are halogen, R⁴ is hydrogen; C₁-C₄-alkyl, it being possible for the alkyl group also to carry one or two C₁-C₄-alkoxy and/or C₁-C₄-alkylthio radicals; C₃-C₇-cycloalkyl; C₃-C₆-alkenyl; C₃-C₆-alkynyl or benzyl and R⁵ is hydrogen or methyl, by reducing a 3-nitrocinnamic ester of the general formula II 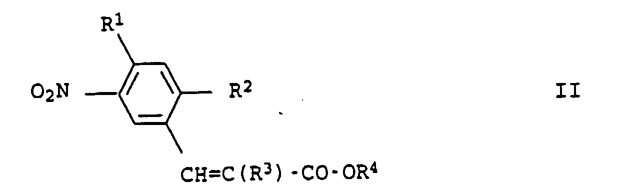 to a 3-aminocinnamic ester of the formula III 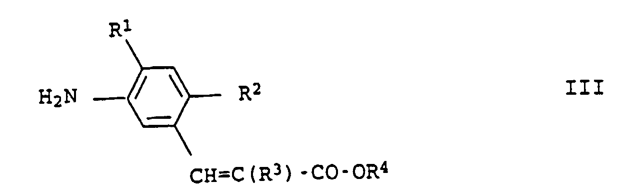 and condensing a 3-aminocinnamic ester of the formula III with a 3,4,5,6-tetrahydrophthalic anhydride of the general formula IV 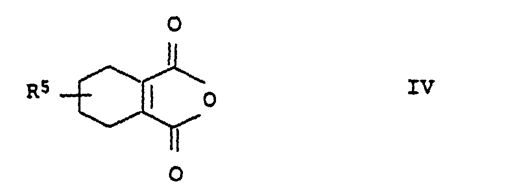 wherein the reduction of the 3-nitrocinnamic ester of the general formula II to the 3-aminocinnamic ester of the general formula III is carried out with hydrogen in the presence of a catalyst from the group of platinum, palladium and nickel, and wherein the 3-aminocinnamic ester of the general formula III is subsequently or simultaneously condensed with a 3,4,5,6-tetrahydrophthalic anhydride of the general formula IV.

2. A process as claimed in claim 1, wherein the 3-aminocinnamic ester of the general formula III is condensed without isolation with a 3,4,5,6-tetrahydrophthalic anhydride of the general formula IV.

3. A process as claimed in claim 1, wherein the reduction is carried out in a polar solvent.

4. A process as claimed in claim 1, which starts from a 3-nitrocinnamic ester II as results in the preparation of II by halogenation of an m-nitrocinnamic ester of the formula VII

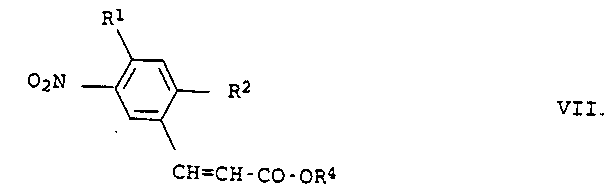

5. A process as claimed in claim 1, which starts from a 3-nitrocinnamic ester II as results in the reaction of an m-nitro aldehyde X 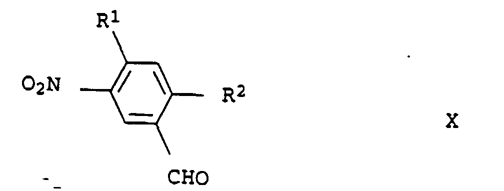 with a phosphorus ylide V
Ar₃P=C(R³)-CO-OR⁴ V.

## Revendications

1. Procédé pour la préparation d'esters 3-(3,4,5,6-tétrahydrophtalimido)cinnamiques de formule générale I 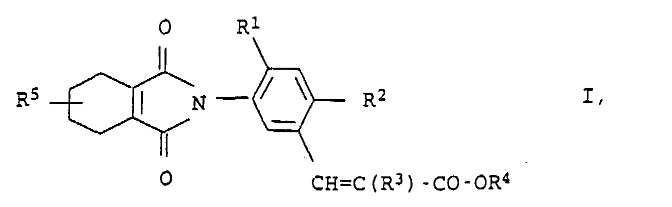 dans laquelle R¹ représente l'hydrogène ou un halogène, R² et R³ des halogènes, R⁴ l'hydrogène ; un groupe alkyle en C1-C4 qui peut encore porter un ou deux groupes alcoxy en C1-C4 et/ou alkylthio en C1-C4 ; un groupe cycloalkyle en C3-C7 ; un groupe alcényle en C3-C6 ; un groupe alcynyle en C3-C6 ou un groupe benzyle, et R⁵ représente l'hydrogène ou un groupe méthyle, dans lequel on réduit un ester 3-nitrocinnamique de formule générale II 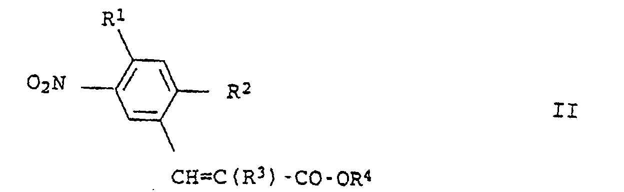 en un ester 3-aminocinnamique de formule III 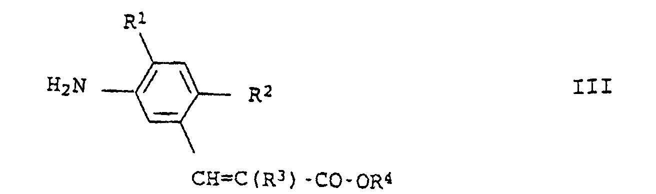 et on condense un ester 3-aminocinnamique de formule III avec un anhydride 3,4,5,6-tétrahydrophtalique de formule générale IV 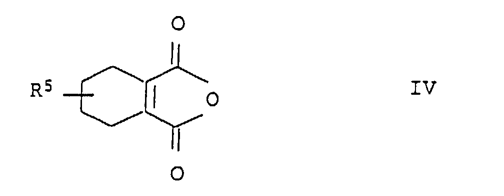 caractérisé par le fait que la réduction de l'ester 3-nitrocinnamique de formule générale II en l'ester 3-aminocinnamique de formule générale III est réalisée à l'aide d'hydrogène en présence d'un catalyseur du groupe du platine, du palladium et du nickel, et que l'ester 3-aminocinnamique de formule générale III est condensé à la suite ou simultanément avec un anhydride 3,4,5,6-tétrahydrophtalique de formule générale IV.

2. Procédé selon la revendication 1, caractérisé par le fait que l'ester 3-aminocinnamique de formule générale III est condensé avec un anhydride 3,4,5,6-tétrahydrophtalique de formule générale IV sans avoir été isolé.

3. Procédé selon la revendication 1, caractérisé par le fait que la réduction est réalisée dans un solvant polaire.

4. Procédé selon la revendication 1, caractérisé par le fait que l'on part d'un ester 3-nitrocinnamique II préparé par halogénation d'un ester m-nitrocinnamique de formule VII

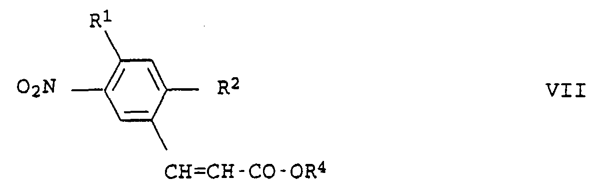

5. Procédé selon la revendication 1, caractérisé par le fait que l'on part d'un ester 3-nitrocinnamique II obtenu par réaction d'un m-nitroaldéhyde X. 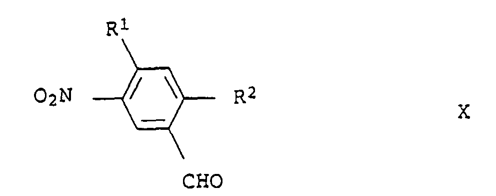 avec un phosphorylide V
Ar₃P=C(R³)-CO-OR⁴ V
